# EUROPEAN PATENT SPECIFICATION

(11) **EP 3 746 074 B1**
(45) Date of publication and mention of the grant of the patent: **20.05.2026**
(21) Application number: 19702265.0
(22) Date of filing: 30.01.2019
(51) Int. Cl.: A61K 31/48, A01K 21/00, A01K 67/00

(54) **COMPOSITION VÉTÉRINAIRE À BASE DE CABERGOLINE UTILISÉE POUR RÉDUIRE LE RETARD DES CHALEURS CHEZ LES TRUIES**
VETERINÄRMEDIZINISCHE CABERGOLIN-ZUSAMMENSETZUNG ZUR REDUZIERUNG DER VERZÖGERTEN BRUNST BEI SAUEN
COMPOSITION VÉTÉRINAIRE DE CABERGOLINE POUR L'UTILISATION A RÉDUIRE L'OESTRUS RETARDÉ CHEZ LES TRUIES

(30) Priority: 30.01.2018 EP 18154264
(43) Date of publication of application: 09.12.2020
(73) Proprietor: Ceva Santé Animale, 33500 LIBOURNE (FR)
(72) Inventor: BERTAIM, Thierry, 33500 LIBOURNE (FR); MANSANET, Camille, 33500 LIBOURNE (FR); ISAKA, Naomie, 33500 LIBOURNE (FR)
(74) Representative: Cabinet Becker et Associés
(86) International application number: PCT/EP2019/052290
(87) International publication number: WO 2019/149775

(56) References cited:
- WO-A1-00/54776
- WO-A1-00/78335
- WO-A1-2009/003965
- KUTZLER ET AL: "Induction and synchronization of estrus in dogs", THERIOGENOLOGY, LOS ALTOS, CA, US, vol. 64, no. 3, 1 August 2005 (2005-08-01), pages 766 - 775, XP027746719, ISSN: 0093-691X, [retrieved on 20050801]
- A. GUNAY ET AL.: "Cabergoline applications in early and late anoestrus periods on German Shepherd dogs", REVUE MÉD. VÉT., vol. 155, no. 11, 1 November 2014 (2014-11-01), pages 557 - 560, XP055484572, Retrieved from the Internet <URL:https://www.revmedvet.com/2004/RMV155_557_560.pdf> [retrieved on 20180614]
- F. DE RENSIS ET AL: "Treatment of lactating sows with the dopamine agonist Cabergoline: effects on LH and prolactin secretion and responses to challenges with naloxone and morphine", ANIMAL REPRODUCTION SCIENCE., vol. 51, no. 3, 1 May 1998 (1998-05-01), NL, pages 233 - 247, XP055484293, ISSN: 0378-4320, DOI: 10.1016/S0378-4320(98)00066-9

## Description

### FIELD OF THE INVENTION

The invention relates to a pharmaceutical or veterinary composition comprising an agonist of antiprolactinic dopamine receptors, for use to improve return to oestrus in porcine, and contributing to oestrus synchronization. It is more precisely related to a method of reduction or prevention of delayed oestrus in sows, comprising administering to said sows, a pharmaceutical or veterinary composition comprising an agonist of antiprolactinic dopamine receptors.The invention is set out in the appended set of claims.

### BACKGROUND OF THE INVENTION

The management of reproduction functions and all biotechnologies associated are key component of the livestock activities, whether in terms of productivity, profitability and of system management. In pig industry, the reproduction management is arduous due to a large variability of oestrus cycle among sows. This variability especially affects the oestrus onset which could be delayed and therefore affects the weaning to oestrus interval (WOI) length, and increase the workload associated to the management of "delayed" sows (regular heat checking and 1 to 3 artificial inseminations). Thus, the improvement of the oestrus cycle control ,i.e the oestrus synchronization by reducing of the WOI length and the prevention of delayed oestrus, could be profitable for porcine farms.

One particularity of pigs farming is the batch management,: each farm building receives animals of same reproductive physiological state. It is therefore of major importance to synchronize sow reproduction , to allow batch conductance management.

Within the context of the invention, "fertility rate" refers to, in case of artificial insemination, the number of farrowing (dead and alive piglets) x100, divided by the number of inseminated females.

The term "oestrus cycle(s)" refers to recurring behavioral, structural and physiological changes occurring between two oestrus, or ovulations, and which are induced by reproductive hormones in females. Oestrus cycles start at the sexual maturity and are interrupted by anoestrus phases in case of fecundation and pregnancy, of lactation or of illness. Oestrus cycles duration in pigs is around 21 days on average.

Oestrus, estrus or estrous, refers to a recurring period of sexual receptivity ("in heat") in female mammals. The female exhibits sexually receptive behavior, a situation that may be signaled by visible physiologic changes. During oestrus, females are receptive to mating. Ovulation occurs spontaneously during oestrus in sows that are neither pregnant nor lactating.

Oestrus is controlled by the hypothalamo-hypophyseal-ovarian axis. The hypothalamus is located in the brain and receives input from external environment and from within the animal's own body and translates them into an hormonal signal. The hormone produced by the hypothalamus in response to environmental stimuli is gonadotropin releasing hormone (GnRH). The hypophysis is located just below the brain and is stimulated by GnRH to produce two hormones, luteinizing hormone (LH) and follicle stimulating hormone(FSH), collectively referred to as gonadotropins. Thus GnRH secreted by the hypothalamus stimulates the secretion of LH and FSH from the pituitary gland. Both LH and FSH act on the ovaries and regulate the growth and the maturity of follicles. As follicles mature, they produce estrogen. Estrogen is the hormone responsible for physiological and behavioral changes normally observed in sows and gilts as they are in oestrus. Moderate levels of estrogen stimulate increased vocalizations, reddening and swelling of the vulva, and the secretion of mucus from the vulva that feels "sticky". High levels of estrogen stimulate the standing reflex or immobilization response (the ultimate sign that confirms a gilt or sow is in oestrus which is in immobilization or "standing" in response to back-pressure from a boar, another gilt or sow), a surge in LH secretion, and consequently ovulation.

Synchronization of oestrus involves controlling the time frame over which this sequence of events takes place in a group of females (batch management). As a result, strategies that are effective for controlling the onset of oestrus manipulate or enhance/inhibit one or more of these physiological events. From a production standpoint, on any given day, it is difficult to predict or control the number of females in oestrus. The ability to precisely control the onset of oestrus is referred to as *synchronization.* In swine industry, reproduction limits come from both the difficulty to predict the onset of oestrus for programming inseminations, and from the difficulty of sows to express oestrus quickly after weaning. In a typical way, the major part of sows expresses oestrus between 4 to 7 days after weaning. However, it is not surprising to observe some sows, particularly primiparous sows, returning to oestrus after more than 7 or 8 days post-weaning, what it is called : "delayed oestrus".

The goal of pigs industries is to increase the litters size and increase the number of litters, to reduce the work load for example by synchronizing cycles without impacting fertility rate. Thus, they use hyper-fertile pig species (cross breeding) and decrease the reproduction time cycles by reducing the WOI. The sow batch management is specific to porcine farming: it improves the work organization, the birth control, and allows to have gathered farrowing and then "homogeneous batches" of piglets at weaning. The duration of a batch cycle is 21 weeks (16 weeks of pregnancy, 4 weeks of suckling, and 1 week of WOI), but one of the goals of livestock farmers is to decrease the 4 weeks of suckling to 3 weeks of suckling in order to increase the productivity.

The weaning is automatically used for the sows synchronization, as it naturally synchronizes animals: it is made in batch on a fixed day for all sows which are not at the same lactation stage, therefore they are not all synchronized.

Nevertheless, synchronization is also obtained by using hormonal treatment: for example altrenogest based progestins treatments (progesterone synthesis agonist: Régumate^{®} or Altresyn^{®}) are used by up to 80% of the livestock farmers to synchronize sows. Farrowing hormonal induction, hormonal stimulation (gonadotropin) or nutritional stimulation ("flushing") of post-weaning sows are also treatments to group inseminations and improve reproduction results: 90% of sows are inseminated with an efficacy of 90%.

More precisely, "oestrus synchronization" is the process of targeting female mammals to come to heat within a short time frame (24 to 96 hours), all together. This is achieved through the use of one or more hormones. GnRH and Prostaglandin F2 are two hormones used in the "Estrus Synch Program" during oestrus synchronization . The synchronization of the oestrus cycle is often used in the dairy and beef industries (and of course in pig industries) in order to decrease the costs for artificial insemination by reducing the period in which it takes for all cows to be in heat and fall pregnant.

Another mean to increase productivity is to reduce the weaning to oestrus interval (WOI) length, in order to reduce the number of non-productive days. When lactating (anoestrus) stops, piglets are weaned and heats come back between 4 and 8 days after: it is called the weaning to oestrus interval (WOI).

The advantages of oestrus synchronization and reduction of WOI length are numerous: a shortened farrowing season provides producers a better opportunity to offer improved management and observation of the animal (sow, cow...) herd, which should result in fewer losses at farrowing, shortened farrowing periods also facilitates improvements in herd health and management such as uniformity in timing of vaccinations and routine management practices resulting in decreased labor requirements. Another benefit is that sow nutrition can be improved by grouping sows according to stage of gestation and feeding each group accordingly. An additional benefit is that the piglets crop will be more uniform in age and size which can lead to an advantage in the market place.

In this goal, research has been conducted into different ways, farmers can perform oestrus synchronization and reduction of WOI length: progesterone injections or a Progesterone Releasing Intra-vaginal Device (PRID^{®}, for bovines). Many hormonal drugs have been developed for sows: Porceptal^{®} (busereline), Fertipig^{®} (gonadotropin), Luteosyl (d-cloprostenol), Dinolyitc^{®} (dinoprost or prostaglandin F2), PG600^{®} (gonadotropin). WO0078335 discloses the use of deslorelin acetate (GnRH) for inducing ovulation in sows or gilts. Nevertheless, even with hormonal treatments, because of the inhibitory effect of suckling and lactation on oestrus onset, because of the suckling differences of each piglet in each sow, because of the fixed same day for the weaning in all sows which are at different lactation stages, returns in the next oestrus cycle remain heterogeneous. Indeed, all sows are not responsive in the same way to hormonal treatments, because their ovaries are not necessarily at the same physiological stage. Thus variabilities between oestrus cycles in sows remain.

Other means are used to shorten the WOI: interrupting suckling, boar exposure, feeding stimulation (lysine...), genetics selection...
Effective management and pharmacological techniques for synchronization of oestrus and to reduce the WOI length in swine are therefore available. However, strategies that work in prepubertal gilts (females under the age of 1 year) are not effective in mature females and vice versa.Moreover, current hormonal treatments with FSH and/or LH activities (as Fertipig^{®}) are used to induce follicular growth and ovulation after weaning at fixed times. However they allow synchronizing heats and ovulations of only sows sensitive to these hormones and able to respond to treatments at the administration time. Furthermore, they do not resolve variability of oestrus cycles between sows until weaning and do not allow preventing delayed oestrus. Finally, these hormonal treatments are forbidden in organic farming. A last mean to increase productivity and fertility rates is to avoid delayed oestrus: the goal is to "gather" the return to heats of all sows and to reduce the number of artificial inseminations. But, currently, there is no treatment to reduce the number of delayed oestrus in order to have an easier reproduction batch conductance management in swine production and a decrease of non-productive days, so less economical losses.

In order to overcome this oestrus onset variability, the applicant has surprisingly discovered that the use of cabergoline, an anti-prolactin dopamine agonist drug, is able to prevent or decrease occurrence of delayed oestrus, when administered to the sows at 2 days before scheduled weaning time.

Dopaminergic agonists are ergot derivatives that inhibit prolactin secretion by stimulating secretion of dopamine or suppressing secretion of serotonin. Consequently, the administration of ergoline derivatives, especially cabergoline, could mimic a synchronized weaning in all sows, independently of the piglets number, their suckling (and environmental parameters) and the different lactation stages of sows, thanks to their administration in a very specific time. By mimicking the weaning, ovaries resume their follicle growth and all sows resume their oestrus cycle. Thus, the post-weaning onset oestrus variability is reduced.

Antiprolactinic compounds are prescribed in medicines for humans to treat hyperprolactinemia, pituitary adenomas, Parkinson disease or even to provoke the stopping of lactation in certain women who cannot breast-feed due to medical reasons. Such medicines contain inhibitors of prolactin hypophyseal as the active principal, a hormone that activates milk secretion. It concerns medicines such as Cabaser^{®}(cabergoline, Pfizer) for which usage is explicitly prohibited during pregnancy because of their deleterious effects on the fetus.

In veterinary medicine, compositions containing cabergoline such as Galastop^{®} (Ceva Santé Animale), or Finilac^{®} (Le Vet) can be further administered as treatments for lactations of pseudo gestations in female cats and dogs. They are also prescribed following early weaning or abortions or during the immediate withdrawal after birth or parturition. In animals, as in human patients, prolactin secreting inhibitors have always been prescribed after gestation or pregnancy because of the toxicity, risks of malformed fetus and the risks of spontaneous abortions.

Dopamine agonists, which inhibit prolactin, successfully induce fertile estrus in most bitches. Prolactin appears to play a part in canine inter estrous intervals, possibly by affecting gonadotropin secretion and/or ovarian responsiveness to gonadotropins. Administration of dopamine agonists shortens the duration of anestrus or induces estrus in cases of prolonged anestrus. But this document deals with bitches, does not disclose the problem of prevention of delayed oestrus and therefore synchronization of oestrus among bitches. It only deals with the technical problem of the oestrus induction.

But none of the current treatments are able to avoid delayed oestrus, to reduce the WOI length, to synchronize the oestrus onset among sows and to treat the seasonal anoestrus in sows.

### SUMMARY OF THE INVENTION

Consequently, in order to overcome the foregoing problems, namely, to obtain a drug which can be efficient to: induce oestrus in sows, synchronize the oestrus onset among sows, prevent delayed oestrus, reduce the WOI length, treat the seasonal anoestrus in sows, improve the next farrowing performances, which avoids the use of hormones which are forbidden in organic farm and not efficient in all sows, which is efficient in all females, there is a need to develop a new composition and to know when and in which quantity to administer this composition.

Therefore, the present invention aims to provide a novel composition for its use to improve reproductive performance and oestrus synchronization in sow, by reducing or preventing delayed oestrus, which can thus reduce the spread of WOI at the level of the sow batch.

Composition, according to the present invention, has numerous advantages compared to prior art. It is safe, not toxic, chemically stable and well accepted (biocompatible). There are no general negative clinical signs due to the use of the composition, and an acceptable general tolerance by the sow. Moreover, even with a dose not very high (about 5µg/kg of body weight), only one single administration (especially intramuscular injection) performed at appropriate timing before weaning, is sufficient.

Besides, the composition according to the present invention has a long half-life, and is well distributed, well metabolized and well eliminated by the body and has a good pharmacokinetic profile in sows. Therefore, the problem solved by the present invention, is to provide a novel pharmaceutical or veterinary efficient composition which overcomes all the previous cited drawbacks, more especially preventing or reducing delayed oestrus, without using any exogenous hormones.

More specifically, the composition according to the invention allows to reduce or prevent the number of delayed oestrus, more specifically to reduce or prevent the number of oestrus on or after 8 days, more preferably on or after 7 days, after weaning, which can thus reduce the spread of WOI at the level of the sow batch.

In a first aspect, the aim of the present invention is to provide a pharmaceutical or veterinary composition comprising cabergoline for use to prevent delayed oestrus in sows, or for use to reduce delayed oestrus in sows.

A further object of the invention is to provide a stable veterinary or pharmaceutical composition comprising cabergoline, medium chain triglycerides and DMSO (dimethyl sulfoxide).

Finally, the invention discloses a method of treatment to prevent or reduce delayed oestrus in sows, comprising administering to said sow, a pharmaceutical or veterinary composition cabergoline.

### DETAILED DESCRIPTION OF THE INVENTION

In a first aspect, this invention relates to a pharmaceutical or veterinary composition comprising cabergoline, for use to prevent or reduce delayed oestrus in sows.

Within the context of invention "pharmaceutical composition" refers to a composition containing drugs used to treat and/or diagnose and/or cure and/or prevent diseases. Furthermore, a drug is any substance or combination of substances (composition) presented as having properties to treat and/or prevent disease(s) in human beings; or any substance or combination of substances which may be used in, or administered to human beings either with a view to restoring, correcting or modifying physiological functions by exerting a pharmacological, immunological or metabolic action, or to making a medical diagnosis (according to the Directive 2004/27/EC).

According to the FDA glossary, within the context of invention "pharmaceutical composition" also refers to a "drug product" which is the finished dosage form that contains a drug substance, generally, but not necessarily in association with other active or inactive ingredients.

A drug is defined as a substance recognized by an official pharmacopoeia or formulary, a substance intended for use in the diagnosis, cure, mitigation, treatment, or prevention of disease, a substance (other than food) intended to affect the structure or any function of the body, a substance intended for use as a component of a medicine but not a device or a component, part or accessory of a device (biological products are included within this definition and are generally covered by the same laws and regulations, but differences exist regarding their manufacturing processes -chemical process versus biological process-).

According to the present invention the term "veterinary" has the same definition as "pharmaceutical", but adapted to animals (meaning non-human beings): "animal" means any living stage of any member of the animal kingdom except human beings. More precisely, a "veterinary drug" (or medicine or composition) means any substance or mixture of substances which is used, or is manufactured, sold or represented as suitable for use, in the diagnosis, treatment, control, eradication, mitigation or prevention of disease or abnormal physical or mental state or the symptoms thereof in an animal; or restoring, correcting, controlling, or modifying any physical, mental or organic function in an animal.

According to the present application, the dopamine receptor agonist is an ergoline or a derivative thereof, and more especially the ergoline is cabergoline.

Ergoline derivatives is a group of chemical compounds whose structural skeleton is the alkaloid ergoline(CAS 478-88-6):

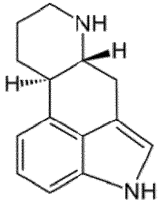

Ergoline derivatives are used clinically to treat vasoconstriction (5-HT₁ receptor agonists, ergotamine) and in the treatment of migraines (used in combination with caffeine) and Parkinson's disease. Some ergoline alkaloids found in ergot fungus (claviceps purpurea) are implicated in the condition ergotism, which causes convulsive and gangrenous symptoms. Other ergoline derivatives are psychedelic substances, including LSD and some alkaloids present in flowering plants such as Argyreia nervosa, Ipomoea tricolor and related species: ergonovine, ergotamine, methergine, pergolide, lisuride...

Another famous ergoline derivative is cabergoline. This a dopamine receptor agonist on D2 receptors. It has a direct inhibitory effect on pituitary lactotroph (prolactin) cells and therefore inhibits prolactin secretion. It is used for the lactation suppression, against hyperprolactinemia, to treat Parkinson's disease and uterine fibroids...

Cabergoline (or 1-[(6-allylergolin-8β-yl)-carbonyl]-1-[3-(dimethylamino)propyl]-3-ethylureaor 1-ethyl-3-(3'-dimethylaminopropyl)-3-(6'-allylergoline-8'β-carbonyl)urea) has the structural formula (CAS 81409-90-7), molecular weight 451.6g.mol⁻¹):

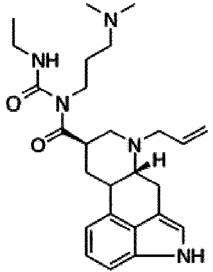

Another object of the invention is a pharmaceutical or veterinary composition comprising cabergoline, as active ingredient, medium chain triglycerides, as an excipient, and DMSO as a solvent.

Medium chain triglycerides are composed of a glycerol backbone whose three hydroxyls are esterified with three fatty acids having an aliphatic tail of 6 to 12 carbon atoms. They can be found in coconut oil, palm kernel oil or butter.

Dimethyl sulfoxide (DMSO, CAS 67-68-5) is an organosulfur compound with the formula S=O(CH₃)₂. This colorless liquid is an aprotic polar solvent that dissolves both nonpolar and polar compounds and is miscible in a wide range of organic solvents as well as water.

This particular composition is used to prevent or reduce delayed oestrus in sows, therefore to reduce the number of sows with a delayed oestrus among a sow batch. In particular, it allows to induce oestrus onset and oestrus synchronization, to improve reproductive performance in sows, reduce the WOI length, reduce the seasonal anoestrus in sows and/or improve the next farrowing performances.

More particularly, the amount of cabergoline, is comprised between about 0.04 and 5 % weight by volume (w/v) of the composition, especially between 0.05 and 4 % w/v, especially between 0.08 and 3 % w/v, especially between 0.09 and 2 % w/v, especially between 0.1 and 1.5 % w/v, especially between 0.15 and 1 % w/v, especially between 0.2 and 0.8 % w/v, especially between 0.05 and 0.5 % w/v, more preferably the cabergoline is present in an amount of 0.11 % w/v of the total composition, more preferably the ergoline derivative (cabergoline preferably) is present in an amount of about0.05 % w/v of the total composition.

More particularly, the amount of medium chain triglycerides is comprised between 85 and 98.96 % weight by volume (w/v) of the composition, especially between 88 and 98 % w/v, especially between 90 and 97% w/v, especially between 92 and 97% w/v, especially between 95 and 97% w/v, especially between 95 and 96.9 % w/v, of the total composition, more preferably the medium chain triglycerides is present in an amount of about 96.88 % w/v of the total composition.

Within the context of invention, % weight/volume or %w/v is the mass concentration defined as the mass (g) of a constituent divided by the volume (100mL) of the total composition.

Furthermore, the composition according to the present invention may further include any of the following other excipients in a pharmaceutically acceptable amount such as, for example, one or more: solvents, antioxidants, flowing agents, lubricants, diluents, preservatives, crystallization inhibitors, colloids, thickeners, thixotropic agents, penetrating agents, stabilizers, solubilizing agents, fluidizing agents, complexing agents, vitamins, minerals, antiseptic agents, or combinations thereof. More generally, the active ingredients may be combined with any liquid additives corresponding to the usual technologies of formulation development.

An excipient, or auxiliary substance, refers to any drug component which is not an active substance (such as adjuvants, stabilizers, diluents, antioxidants, antimicrobial preservatives...), according to pharmacopeias.

Solvents (polar, apolar, protic, aprotic) may be selected from: benzylalcohol, N-octyl-2-pyrrolidone (NOP), N-methyl-2-pyrrolidone (NMP), propylene carbonate, transcutol P or HP^{®} (2-(2-ethoxyethoxy)ethanol or highly purified diethylene glycol monoethyl ether), acetone, 2-butanone, 3-methyl-2-butanone, cyclohexanone, acetonitrile, xylene, chlorobenzene, methylene chloride, chloroform trichloroethane, benzaldehyde ethylene chloride, sulfolane, methyl tert-butyl ether, dibutyl ether, ethyl acetate, acetate propyl methacrylate, amyl acetate, propyl acetate, dimethylformamide (DMF), dimethylacetamide (DMAC), propylene diethylcarbonate, ethylene carbonate, acetonitrile, triethylamine, pyridine, methanol, ethanol, isopropanol, hexafluoroisopropanol, carboxylic acids such as formic acid and acetic acid, primary and secondary amines, propylene alkyl ether, ethylene alkyl ether, polyglycol alkyl ether, di polyglycol allyl, polypropylene glycol, polyethylene glycol... and mixtures thereof. The preferred solvents are benzylalcohol, DMSO, NOP, NMP, propylene carbonate, and/or transcutol P or HP ^{®} (2-(2-ethoxyethoxy)ethanol or highly purified diethylene glycol monoethyl ether). In a particular embodiment, the composition comprises a solvent such as DMSO. The solvent or the mixture of solvents are present in an amount of between1 % w/v and 10 % w/v of the total composition, more preferably is present in an amount between 2 % w/v and 5 % w/v of the total composition, more preferably is present in an amount of 3 % w/v of the total composition.

Antioxidants, when added, may be selected from: 2,6-di-tert-butyl-4-methylphenol (butyl hydroxytoluene or BHT), vitamin E (DL-alpha-tocopherol, E307), vitamin E phosphate, vitamin A, ascorbic acid (vitamin C), vitamin B12, polyphenols, butyl hydroxyanisol (BHA), propylgallate, tocopherol, ascorbic acid, citric acid, di-alpha-tocopheryl phosphate, beta-carotene, carotenes, carotenoids, flavonoids, sulfate compounds, L-cysteine, thiodipropionic acid, thiolactic acid, monothioglycerol, propyl galate sodium metabisulfite, sodium formaldehyde, sulfoxylate acetate, and mixtures thereof. The preferred antioxidant is BHT. Antioxidants/antioxidant are/is present in an amount of between 0.001 and 2% w/v.

Preservatives, when added, may be selected from: methyl p-hydroxybenzoate, ethyl p-hydroxybenzoate, phenol, sorbic acid, cresol and chlorocresol, and mixtures thereof... Preservatives are present in an amount of 0,001-2% w/v of the composition.

Illustrative thickening agents, when added, include methylcellulose, hydroxyethyl cellulose, sodium carboxymethyl cellulose, hydroxypropyl cellulose, polyvinylpyrrolidone, and mixtures thereof.

Illustrative complexing agents, when added, include EDTA and salts thereof, phosphate, nitrate, acetate, citrate, and mixtures thereof.

Illustrative antiseptics, when added, include methyl p-oxybenzoate, propyl p-oxybenzoate, PHB ester, chlorobutanol, benzyl alcohol, butanol, butane-1,3-diol, chlorohexidin salts, benzoic acid and its salts, sorbic acid, and mixtures thereof.

According to the present invention, sow(s) refer(s) to the females of Sus scrofa domesticus, which include mature sows and young gilts. In this application, guilt or sow can be used independently. The sow can be a multiparous sow (having experienced one or more previous parturitions) or a primiparous sow (a guilt/sow bearing a first offspring). More especially, sows are primiparous sows.

In a particular embodiment, the composition is administered via an injection, more precisely an intramuscular injection -which means directly into muscle-, in one single administration.

In a further embodiment, the composition according to the present application is administered to the sow two days before, or one day before, more especially, between about two days and about one day before the piglet weaning day.

In another embodiment, the composition according to the present application comprises ergoline, more especially cabergoline, in a therapeutic dose comprised of about 2 µg/kg of body weight and about 7 µg/kg of body weight, more especially about 5.6 µg/kg of body weight, even more especially about 5 µg/kg of body weight.

In the present application, the term "about" includes all values within a range of 10% of the stated number (above or below the numerical value), especially all values within a range of 5% of the stated number, especially all values within a range of 3% of the stated number, more especially all values within a range of 2% of the stated number, and even more especially all values within a range of 1% of the stated number.

The composition according to the invention is in the form of a liquid solution, semi-liquid solution, or suspension.

In a further embodiment, the composition according to the present application is characterized in that the reduction and/or prevention of delayed oestrus implies a shorter WOI with a smaller range of dispersion and contribute to the oestrus synchronization.

Within the context of the invention "oestrus synchronization" refers to the ability to gather the onset of oestrus, among sows, gather the time frame over which the heats come back in a group of females. It is the process of targeting female mammals to come to heat within a short time frame, all together. A preferred time frame is inferior or equal to 6 days after weaning, especially from3 days to 6 days, more especially from 3 to 5 days, and more preferably from 3 to 4 days.

In a further embodiment of the invention, piglets weaning is set between 3 weeks (about 18 days to 24 days) after lactation and 4 weeks (about 25 days to 31 days) after lactation.

Within the context of invention "delayed oestrus" refers to sows/guilts which return to heat after the majority of sows, not matching with them (for example 8 or 9 days after weaning). More specifically, it includes sows/guilts which return to oestrus after more than 7 or 8 days post-weaning.

Composition is formulated as a unit dose adapted to the weight of the animal, and the entire dose is administered to the animal. Thanks to the injectable application method, the amount of cabergoline in sows is known and controlled.

Another object of the present invention is a method of treatment to prevent or reduce delayed oestrus in sows, comprising administering to said sow, a pharmaceutical or veterinary composition comprising cabergoline.

All embodiments described above for the composition also apply to the use of said composition and to the method of treatment as described below.

Described herein below are examples: preparation of compositions according to the present invention and efficiency tests. These examples are illustrative and in no way limiting.

### EXAMPLES

Example 1 :Preparation of 9L of an injectable solution containing 500 µg/mL of cabergoline.

### Formula:

- Active substance : 4.53 g of cabergoline (titrating 100 %)
- Excipient: triglycerides of a medium chain QS 9L.
   1) Step 1: 4.53 g of cabergoline and 1.5 kg medium chain triglycerides were measured in a recipient of adequate capacity, and then a mix was shaken with a magnetic shaker (500 turns per minute) for at least 60 minutes for complete dissolving.
   2) Step 2: In a dry recipient, 5 kg medium chain triglycerides were measured, the flow of nitrogen and the mixer were set and then the cabergoline in a concentrated solution, obtained at step 1, was added to the recipient. The shaking was maintained for 30 minutes and then the volume was brought to a final volume of 9 liters by adding medium chain triglycerides. The shaking was still maintained for another 30 minutes and then solution was filtered under pressurized nitrogen through a calibrated cartridge of 0.45 microns. The filtrate was collected in an appropriate flask previously disinfected by vapor and dried with a flow of nitrogen. The solution obtained was sterilized at 121 °C during minutes, and then was put into sterilized, non-pyrogenic flasks shut with rubber stoppers and aluminum capsules, washed and passed through the autoclave.

### Example 2: Veterinary injectable composition according to the present application

| | |
|---|---|
| Cabergoline | 112 mg |
| DMSO | 3000 mg |
| Medium chain triglycerides | QS 100mL |

### Example 3 :Proof of concept study, WOI

All sows included in this study were recruited from a single farm having an historical mean of WOI of 5.4 days, and a percentage of delayed oestrus (≥8 days) of 7.9%. Weaning was set after about 3 weeks of lactation.

Primiparous sows (with normal health status and normal farrowing) were allocated into three groups:
- Cab_0 (n=19 sows): 5.6µg/kg of cabergoline by intramuscular injection at weaning day (day 0) in sows, i.e. at the end of lactation, without suckling piglets.
- Cab_2 (n=30 sows): 5.6µg/kg of cabergoline by intramuscular injection 2 days before weaning (day - 2) in sows, i.e. lactation still pending with suckling piglets.
- Negative control group (n=29 sows): equivalent dose/volume of a 'true' placebo by intramuscular injection at Day-2 and day 0.

Distributions of individual WOI by groups are given here after:

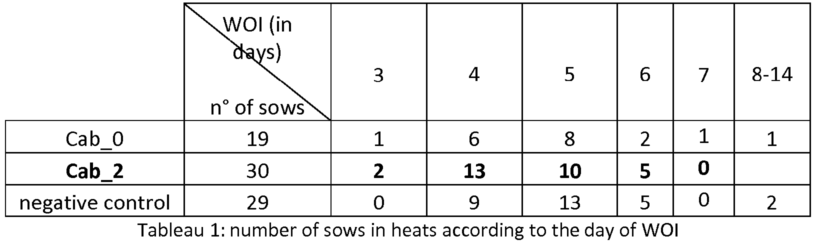

In the negative group, 2/29 (6.9%) of the sows exhibited a delayed oestrus, i.e an oestrus occurring after or equal 8 days, whereas no delayed oestrus was observed in Cab_2 group. In Cab_0 group 1/19 (5.3%) of sows exhibited delayed oestrus after or equal 8 days. Cab_0 and Cab_2 groups showed some quick heat returns at day 3, in contrast with placebo group. In Cab_2, there is no delayed oestrus, and the WOI spreads between days 3 and 6 only, instead of days 3 to 14 in Cab_0 and placebo groups.

All sows were diagnosed positively pregnant after artificial insemination following oestrus detection. There was thus no negative impact of the treatment on fertility.

## Claims

1. A pharmaceutical or veterinary composition comprising cabergoline, for use to reduce delayed oestrus in sows.

2. The pharmaceutical or veterinary composition according to claim 1, wherein the composition is administered to the sow between about two days and about one day before the piglets weaning day.

3. The pharmaceutical or veterinary composition for use according to any of the claims 1-4, wherein the composition is administered via intramuscular injection.

4. The pharmaceutical or veterinary composition for use according to any of the claims 1-5, wherein the composition is administered in one single administration.

5. The pharmaceutical or veterinary composition for use according to any of the claims 1-6, wherein the ergoline is present in a therapeutic dose of about 5 µg/kg of body weight.

6. The pharmaceutical or veterinary composition for use according to any of the claims 1-7, wherein the sow is a primiparous sow.

7. The pharmaceutical or veterinary composition for use according to any of the claims 1-7, wherein the sow is a multiparous sow.

8. The pharmaceutical or veterinary composition for use according to any of the claims 1-9, comprising cabergoline, medium chain triglycerides and DMSO.

9. A non-therapeutic method to reduce or prevent delayed oestrus in sows, comprising administering to said sow, a pharmaceutical or veterinary composition comprising cabergoline.

## Patentansprüche

1. Pharmazeutische oder veterinäre Zusammensetzung, umfassend Cabergolin, zur Verwendung, um verzögerten Östrus bei Sauen zu reduzieren.

2. Pharmazeutische oder veterinäre Zusammensetzung nach Anspruch 1, wobei die Zusammensetzung der Sau zwischen etwa zwei Tagen und etwa einem Tag vor dem Absetzungstag der Ferkel verabreicht wird.

3. Pharmazeutische oder veterinäre Zusammensetzung zur Verwendung nach einem der Ansprüche 1 bis 4, wobei die Zusammensetzung über intramuskuläre Injektion verabreicht wird.

4. Pharmazeutische oder veterinäre Zusammensetzung zur Verwendung nach einem der Ansprüche 1 bis 5, wobei die Zusammensetzung in einer einzigen Verabreichung verabreicht wird.

5. Pharmazeutische oder veterinäre Zusammensetzung zur Verwendung nach einem der Ansprüche 1 bis 6, wobei das Ergolin in einer therapeutischen Dosis von etwa 5 µg/kg Körpergewicht vorliegt.

6. Pharmazeutische oder veterinäre Zusammensetzung zur Verwendung nach einem der Ansprüche 1 bis 7, wobei die Sau eine erstgebärende Sau ist.

7. Pharmazeutische oder veterinäre Zusammensetzung zur Verwendung nach einem der Ansprüche 1 bis 7, wobei die Sau eine mehrgebärende Sau ist.

8. Pharmazeutische oder veterinäre Zusammensetzung zur Verwendung nach einem der Ansprüche 1 bis 9, umfassend Cabergolin, mittelkettige Triglyceride und DMSO.

9. Nicht-therapeutisches Verfahren, um verzögerten Östrus bei Sauen zu reduzieren oder zu verhindern, umfassend das Verabreichen einer pharmazeutischen oder veterinären Zusammensetzung, umfassend Cabergolin, an die Sau.

## Revendications

1. Composition pharmaceutique ou vétérinaire comprenant de la cabergoline, pour utilisation afin de réduire le retard d'oestrus chez des truies.

2. Composition pharmaceutique ou vétérinaire selon la revendication 1, dans laquelle la composition est administrée à la truie entre environ deux jours et environ un jour avant le jour de sevrage des porcelets.

3. Composition pharmaceutique ou vétérinaire pour utilisation selon l'une quelconque des revendications 1 à 4, dans laquelle la composition est administrée par injection intramusculaire.

4. Composition pharmaceutique ou vétérinaire pour utilisation selon l'une quelconque des revendications 1 à 5, dans laquelle la composition est administrée en une seule administration.

5. Composition pharmaceutique ou vétérinaire pour utilisation selon l'une quelconque des revendications 1 à 6, dans laquelle l'ergoline est présente dans une dose thérapeutique d'environ 5 µg/kg de poids corporel.

6. Composition pharmaceutique ou vétérinaire pour utilisation selon l'une quelconque des revendications 1 à 7, dans laquelle la truie est une truie primipare.

7. Composition pharmaceutique ou vétérinaire pour utilisation selon l'une quelconque des revendications 1 à 7, dans laquelle la truie est une truie multipare.

8. Composition pharmaceutique ou vétérinaire pour utilisation selon l'une quelconque des revendications 1 à 9, comprenant de la cabergoline, des triglycérides à chaîne moyenne et du DMSO.

9. Procédé non thérapeutique permettant de réduire ou prévenir un œstrus retardé chez des truies, comprenant l'administration à ladite truie, d'une composition pharmaceutique ou vétérinaire comprenant de la cabergoline.
